# EUROPEAN PATENT APPLICATION

(11) **EP 4 403 159 A1**
(43) Date of publication of application: **24.07.2024**
(21) Application number: 22870060.5
(22) Date of filing: 16.09.2022
(51) Int. Cl.: A61K 6/30, A61K 6/58, A61K 6/62, A61K 6/887

(54) **METHOD FOR PRODUCING DENTURE**

(30) Priority: 17.09.2021 JP 2021152634
(71) Applicant: Kuraray Noritake Dental Inc., Kurashiki-shi, Okayama 710-0801 (JP)
(72) Inventor: SUZUKI, Kenji, Tainai-shi, Niigata 959-2653 (JP)
(74) Representative: D Young & Co LLP
(86) International application number: PCT/JP2022/034823
(87) International publication number: WO 2023/042913

(57) **Abstract**

The present invention relates to a method of producing a denture, including adhering a denture base that is a stereolithographic article of a curable composition (I) containing a (meth)acrylic based polymerizable monomer component (A) containing a (meth)acrylic based polymerizable monomer (m) as a major monomer, and a photopolymerization initiator, and a prosthetic tooth containing an acrylic based resin (II), with an adhesive composition containing a composition (III) containing a (meth)acrylic based polymerizable monomer component (C) containing the same (meth)acrylic based polymerizable monomer (m) as contained in the curable composition (I).

## Description

### Technical Field

The present invention relates to a method of producing a denture including adhering a denture base produced by stereolithography and a prosthetic tooth by using a particular adhesive composition.

### Background Art

Dentures have been produced by the lost-wax process shown below. First, a mold of the oral cavity of the patient is prepared, a base plate is formed on a gypsum model produced based on the mold, and a gingival part formed of wax, which is referred to as a wax rim, is further built thereon to produce a biteplate. Next, after bite taking, prosthetic teeth formed of an acrylic resin are arranged, and a wax denture is completed through occlusal adjustment and gingival formation. Subsequently, after performing an operation referred to as embedding, i.e., embedding the wax denture and the gypsum model in a flame, which is referred to as a flask, with gypsum, the wax is melted and removed with hot water, the plate is also removed, and then an acrylic resin is poured into the cavity and then cured, followed by performing an operation for finishing, thereby completing the denture. This method not only includes a large number of process steps, but also requires extremely high dimensional accuracy on the order of several micrometers for the denture production, requiring not only a large amount of time but also the skill of the dental mechanic.

In recent years, CAD/CAM systems that designs dentures on a screen using a computer and produces dentures by cutting from a block material formed of a resin or ceramics are attracting attention as a method capable of stably providing dentures of a certain quality within a short period of time, and the denture designing and production systems, such as Cerec System, have become widespread. Among these, the system using a stereolithographic 3D printer is rapidly spreading.

In the stereolithographic method, a base and a prosthetic tooth part are separately produced, and therefore are necessarily adhered after the production thereof. In the stereolithographic method, however, a polymerizable compound having particularly high curability is necessarily used since the compound is instantaneously cured with light, and therefore a crosslinkable monomer and a monomer exhibiting high cohesive force are used. Therefore, a denture base material produced by stereolithography has a high crosslinking density and a high cohesive force. In general, an adhesive formed of methyl methacrylate (MMA) having a polymerization initiator mixed therein and acrylic resin powder is used for adhering a prosthetic tooth and a base for restoration or the like, but the adhesive is difficult to penetrate to the base produced by stereolithography, which results in a problem that a sufficient adhesion force cannot be obtained.

The dental adhesive materials (adhesive compositions) having been widely known include an adhesive applied to the dentine, an adhesive applied to a dental composite resin, and also an adhesive applied to a dental prosthesis formed of a metal or ceramics. The adhesive materials that target these applications contain materials that form chemical bonds to the adherends, such as an acidic monomer, a silane coupling agent, and a sulfur-containing monomer, but the adhesive material applied to a denture base material has a mechanism exhibiting adhesiveness that is completely different from the adhesives for these applications.

In view of the background described above, PTL 1 describes a production example of a denture obtained by adhering a denture base produced by using a CAD/CAM system and a prosthetic tooth formed of an acrylic resin with a powder-liquid type resin containing MMA and PMMA.

### Citation List

### Patent Literature

PTL 1: WO2018/159507

### Summary of Invention

### Technical Problem

However, PTL 1 is an example that the base is designed to a particular shape, and fails to describe the adhesiveness between a denture base produced by stereolithography and a prosthetic tooth.

Under the circumstances, an object of the present invention is to provide a method of producing a denture including firmly adhering a denture base produced by stereolithography and a prosthetic tooth containing an acrylic based resin.

### Solution to Problem

The present invention encompasses the following inventions.
[1] A method of producing a denture, including adhering
   a denture base that is a stereolithographic article of a curable composition (I) containing a (meth)acrylic based polymerizable monomer component (A) containing a (meth)acrylic based polymerizable monomer (m) as a major monomer, and a photopolymerization initiator, and a prosthetic tooth containing an acrylic based resin (II),
   with an adhesive composition containing a composition (III) containing a (meth)acrylic based polymerizable monomer component (C) containing the same (meth)acrylic based polymerizable monomer (m) as contained in the curable composition (I).
[2] The method of producing a denture according to the item [1], in which the (meth)acrylic based polymerizable monomer (m) is a polyfunctional (meth)acrylic based polymerizable monomer (a-1).
[3] The method of producing a denture according to the item [2], in which the polyfunctional (meth)acrylic based polymerizable monomer (a-1) is at least one kind selected from a urethanated (meth)acrylate ester compound and an aromatic compound based (meth)acrylate ester compound having no urethane bond.
[4] The method of producing a denture according to any one of the items [1] to [3], in which the (meth)acrylic based polymerizable monomer component (A) contains a monofunctional (meth)acrylic based polymerizable monomer (a-2).
[5] The method of producing a denture according to any one of the items [1] to [4], in which the (meth)acrylic based polymerizable monomer component (C) contains a monofunctional (meth)acrylic based polymerizable monomer (a-2).
[6] The method of producing a denture according to the item [4] or [5], in which the monofunctional (meth)acrylic based polymerizable monomer (a-2) is a (meth)acrylate ester based polymerizable monomer having an aromatic ring.
[7] The method of producing a denture according to any one of the items [1] to [6], in which the curable composition (I) has a content of the (meth)acrylic based polymerizable monomer (m) of 50% by mass or more based on 100% by mass of the (meth)acrylic based polymerizable monomer component (A).
[8] The method of producing a denture according to any one of the items [1] to [7], in which the composition (III) has a content of the (meth)acrylic based polymerizable monomer (m) of 50% by mass or more based on 100% by mass of the (meth)acrylic based polymerizable monomer component (C).
[9] The method of producing a denture according to any one of the items [1] to [8], in which the curable composition (I) contains the (meth)acrylic based polymerizable monomer component (A) in an amount of 40% by mass or more based on 100% by mass of the curable composition (I).
[10] The method of producing a denture according to any one of the items [1] to [9], in which the composition (III) contains the (meth)acrylic based polymerizable monomer (C) in an amount of 40% by mass or more based on 100% by mass of the composition (III).
[11] The method of producing a denture according to any one of the items [1] to [10], in which the method includes the following steps (1) and (2) in this order:
   step (1): a step of coating the adhesive composition on at least one selected from the denture base and the prosthetic tooth, and
   step (2): a step of adhering the denture base and the prosthetic tooth via the adhesive composition under pressure.
[12] The method of producing a denture according to any one of the items [1] to [11], in which the method includes irradiating the adhesive composition with an active energy light ray, so that the adhesive composition is cured to adhere the denture base and the prosthetic tooth.

### Advantageous Effects of Invention

The present invention can provide a method of producing a denture including firmly adhering a denture base produced by stereolithography and a prosthetic tooth containing an acrylic based resin.

### Description of Embodiments

The present invention will be described in detail with reference to embodiments below.

In the description herein, the upper limit values and the lower limit values of the numeral ranges (such as the contents of the components, the values calculated from the components, and the property values) can be appropriately combined.

Therefore, in the description herein, the lower limit values and the upper limit values described in a stepwise manner for the numeral ranges of the same item can be independently combined. For example, based on the description "preferably 10 to 90, and more preferably 30 to 60" for the same item, the "preferred lower limit value (10)" and the "more preferred upper limit value (60)" can be combined to make a range of "10 to 60".

As for the numeral ranges, for example, based on the description "preferably 10 to 90, and more preferably 30 to 60", only the lower limit value can be defined as "10 or more" or "30 or more" without particularly limiting the upper limit value. Similarly, only the upper limit value can be defined as "90 or less" or "60 or less" without particularly limiting the lower limit value.

Unless otherwise indicated, the description "10 to 90" simply for the numeral range means a range of 10 or more and 90 or less.

As similar to the above, for example, based on the description "preferably 10 or more, and more preferably 30 or more" and the description "preferably 90 or less, and more preferably 60 or less" for the same item, the "preferred lower limit value (10)" and the "more preferred upper limit value (60)" can be combined to make a range of "10 or more and 60 or less". As similar to the above, only the lower limit value can be defined as " 10 or more" or "30 or more", and similarly, only the upper limit value can be defined as "90 or less" or "60 or less".

In the description herein, the expression "(meth)acrylic" is used as the concept encompassing both "methacrylic" or "acrylic". This is similarly applied to the analogous expressions including "(meth)acrylate", "(meth)acrylamide", "(meth)acryloyloxy", and the like.

In the description herein, unless otherwise indicated, the terms having the same symbol (for example, the "composition (I)", the "acrylic based resin (II)", the "composition (III)", the "monomer (m)", the "monomer (o)", the "monomer (a-1)", the "monomer (a-2)", the "component (A)", and the "component (C)") indicate the same item.

In the description herein, unless otherwise indicated, the description of "adhesiveness" means the adhesiveness between the denture base and the prosthetic tooth.

### [Method of producing Denture]

The method of producing a denture as one embodiment of the present invention includes adhering a denture base that is a stereolithographic article of a curable composition (I) containing a (meth)acrylic based polymerizable monomer component (A) containing a (meth)acrylic based polymerizable monomer (m) as a major monomer, and a photopolymerization initiator, and a prosthetic tooth containing an acrylic based resin (II), with an adhesive composition containing a composition (III) containing a (meth)acrylic based polymerizable monomer component (C) containing the same (meth)acrylic based polymerizable monomer (m) as contained in the curable composition (I).

It is estimated that the use of the adhesive composition containing a composition (III) containing a (meth)acrylic based polymerizable monomer component (C) containing the same (meth)acrylic based polymerizable monomer (m) as contained in the curable composition (I) for the denture base that is a stereolithographic article of the curable composition (I) facilitates the penetration of the adhesive composition containing the composition (III) to the surface of the denture base produced from the curable composition (I) by stereolithography, and the interaction of the same (meth)acrylic based polymerizable monomer (m) contained in the curable composition (I) and the composition (III), for example, hydrogen bond, hydrophobic interaction, and π-electron interaction, can be obtained to achieve further firm adhesiveness.

It is also estimated that the composition (III) has affinity with the acrylic based resin (II), and thereby can further firmly adhere the denture base that is a stereolithographic article of the curable composition (I) and the prosthetic tooth containing the acrylic based resin (II).

The components that can be used in the method of producing a denture as one embodiment of the present invention will be described below.

### [Curable Composition (I)]

The curable composition (I) (which may be hereinafter referred simply to as a "composition (I)") contains a (meth)acrylic based polymerizable monomer component (A) (which may be hereinafter referred simply to as a "component (A)") containing a (meth)acrylic based polymerizable monomer (m) (which may be hereinafter referred simply to as a "monomer (m)") as a major monomer, and a photopolymerization initiator. The composition (I) has photocurability.

### <(Meth)acrylic based Polymerizable Monomer Component (A)>

The (meth)acrylic based polymerizable monomer component (A) is a component that is constituted by one kind of the (meth)acrylic based polymerizable monomer alone, or by two or more kinds thereof, and contains a (meth)acrylic based polymerizable monomer (m) as a major monomer.

In the description herein, the "major monomer" means the (meth)acrylic based polymerizable monomer that has the largest content among the (meth)acrylic based polymerizable monomers constituting the component (A).

In the description herein, the "(meth)acrylic based polymerizable monomer component" means a component that is constituted by one kind of the (meth)acrylic based polymerizable monomer alone, or by two or more kinds thereof, which is applied to the component (A), and similarly to the "(meth)acrylic based polymerizable monomer component (C)" described later.

In the description herein, the "(meth)acrylic based polymerizable monomer" means one specific compound constituting the "(meth)acrylic based polymerizable monomer component", i.e., a particular (meth)acrylic based polymerizable monomer, which is applied to the monomer (m), and similarly to the "polyfunctional (meth)acrylic based polymerizable monomer (a-1)" and the "monofunctional (meth)acrylic based polymerizable monomer (a-2)" described later.

The content of the monomer (m) contained as the major monomer in the component (A) is the largest in the component (A), is not particularly limited, as far as being the content of a (meth)acrylic based polymerizable monomer (o) (which may be hereinafter referred simply to as a "monomer (o)") other than the monomer (m) or more, and for example, is preferably 20% by mass or more, more preferably 30% by mass or more, further preferably 40% by mass or more, still further preferably 50% by mass or more, still more further preferably 55% by mass or more, even further preferably 60% by mass or more, and even still further preferably 65% by mass or more, and for example, is 100% by mass or less, preferably 98% by mass or less, more preferably 95% by mass or less, further preferably 90% by mass or less, still further preferably 85% by mass or less, and still more further preferably 80% by mass or less, based on 100% by mass of the component (A). As described above, the lower limit values and the upper limit values described in a stepwise manner can be independently combined. For example, in one embodiment of the present invention, the content of the monomer (m) in the component (A) is preferably 20 to 100% by mass, more preferably 30 to 100% by mass, further preferably 40 to 98% by mass, still further preferably 50 to 95% by mass, still more further preferably 55 to 90% by mass, even further preferably 60 to 85% by mass, and even still further preferably 65 to 80% by mass, based on 100% by mass of the component (A).

As described above, the component (A) is a component that is constituted only by the monomer (m) alone, or by the monomer (m) and the monomer (o). The component (A) may contain multiple kinds of the monomer (o). Therefore, the total content of the monomer (m) and the monomer (o) in the component (A) is 100% by mass.

### ((Meth)acrylic based Polymerizable Monomer)

Examples of the (meth)acrylic based polymerizable monomer constituting the component (A) include a (meth)acrylate ester based polymerizable monomer and a (meth)acrylamide based polymerizable monomer.

Examples of the (meth)acrylic based polymerizable monomer include a polyfunctional (meth)acrylic based polymerizable monomer (a-1) having multiple polymerizable groups (which may be hereinafter referred simply to as a "polyfunctional monomer (a-1)") and a monofunctional (meth)acrylic based polymerizable monomer (a-2) having one polymerizable group (which may be hereinafter referred simply to as a "monofunctional monomer (a-2)").

From the standpoint of achieving more excellent adhesiveness, it is preferred that the component (A) contains the polyfunctional monomer (a-1), and it is more preferred that the (meth)acrylic based polymerizable monomer (m) contained as a main monomer is the polyfunctional monomer (a-1).

It is preferred that the component (A) contains the monofunctional monomer (a-2), and from the standpoint of facilitating the excellent curability of the composition (I), and facilitating the penetration of the adhesive described later to the denture base, for further facilitating the excellent adhesiveness, it is more preferred that the component (A) contains both the polyfunctional monomer (a-1) and the monofunctional monomer (a-2), and it is further preferred that the component (A) contains the polyfunctional monomer (a-1) as a major monomer and further contains the monofunctional monomer (a-2). In the case where the component (C) described later contains the monomer (a-2), the preferred ranges therefor are the same as above from the standpoint of achieving the excellent curability of the composition (III), and facilitating the penetration of the adhesive composition to the denture base and the prosthetic tooth, for more easily exhibiting the excellent adhesiveness.

The component (A) may contain one kind of the polyfunctional monomer (a-1) alone, or may contain two or more kinds thereof, and similarly may contain one kind of the monofunctional monomer (a-2) alone, or may contain two or more kinds thereof.

### [Polyfunctional (Meth)acrylic based Polymerizable Monomer (a-1)]

The polyfunctional monomer (a-1) is preferably at least one kind selected from the group consisting of a urethanated (meth)acrylate ester compound, an aromatic compound based (meth)acrylate ester compound having no urethane bond, and an alicyclic (meth)acrylate ester compound, more preferably at least one kind selected from a urethanated (meth)acrylate ester compound and an aromatic compound based (meth)acrylate ester compound having no urethane bond, and further preferably a urethanated (meth)acrylate ester compound, from the standpoint of enhancing the curability of the composition (I) and enhancing the adhesiveness. In other words, the component (A) preferably contains at least one kind selected from the group consisting of a urethanated (meth)acrylate ester compound, an aromatic compound based (meth)acrylate ester compound having no urethane bond, and an alicyclic (meth)acrylate ester compound, more preferably contains at least one kind selected from a urethanated (meth)acrylate ester compound and an aromatic compound based (meth)acrylate ester compound having no urethane bond, and further preferably contains a urethanated (meth)acrylate ester compound, as the polyfunctional monomer (a-1) described above..

As described above, the component (A) preferably contains at least one kind selected from the group consisting of a urethanated (meth)acrylate ester compound, an aromatic compound based (meth)acrylate ester compound having no urethane bond, and an alicyclic (meth)acrylate ester compound, which each are the polyfunctional monomer (a-1), more preferably contains at least one kind selected from a urethanated (meth)acrylate ester compound and an aromatic compound based (meth)acrylate ester compound having no urethane bond, and further preferably contains a urethanated (meth)acrylate ester compound, as the (meth)acrylic based polymerizable monomer (m) contained as the major monomer.

The aromatic compound based (meth)acrylate ester compound having no urethane bond more preferably contains at least one kind selected from the group consisting of a hydroxy group, a primary amino group, and a secondary amino group.

It is estimated that these compounds not only accelerate the curing through the dielectric effect of the urethane bond, the aromatic skeleton, the hydroxy group, the primary amino group, and the secondary amino group, but also form a chemical bond, such as a hydrogen bond and a π-electron effect. Among these, the urethanated (meth)acrylate ester compound having a urethane bond is preferred since the compound has a large effect of imparting toughness to the denture, and thereby breakage and cracks are difficult to occur at the adhesion interface and the coated film, and the resulting denture can exhibit a further excellent flexural strength.

### {Urethanated (Meth)acrylate Ester Compound}

The urethanated (meth)acrylate ester compound can be easily synthesized, for example, by subjecting a compound containing one or more kind selected from an alkylene skeleton and a phenylene skeleton and having an isocyanate group, and a (meth)acrylate compound having a hydroxy group (-OH), to addition reaction.

The weight average molecular weight of the urethanated (meth)acrylate ester compound is preferably 1,000 or less from the standpoint of facilitating forming of the composition (I) due to low viscosity thereof and facilitating the penetration of the adhesive composition described later. This is also applied to the case where the component (C) described later contains the urethanated (meth)acrylate ester compound from the standpoint that the adhesive composition can be easily coated due to low viscosity, and the penetration of the adhesive composition to the denture base and the prosthetic tooth can be easily obtained. The weight average molecular weight is more preferably 750 or less, and further preferably 500 or less, from the standpoints described above. The weight average molecular weight referred herein means a polystyrene conversion weight average molecular weight obtained by gel permeation chromatography (GPC). In the case where the urethanated (meth)acrylate ester compound does not contain a polymer structure, there is no concept of weight average molecular weight, and therefore the molecular weight thereof is applied.

The urethanated (meth)acrylate ester compound is preferably a compound that does not contain a polymer structure, such as polyester, polycarbonate, polyurethane, and polyether. The absence of the structure contained can prevent the concentration of the functional group having polarity from being increased, and can prevent the decrease of the adhesiveness in the presence of water, for example, in the oral cavity. In particular, the number of the urethane bond is preferably 3 or less, and more preferably 2 or less, per one molecule, from the standpoint of the adhesiveness in the presence of water. In other words, the number of the urethane bond in the urethanated (meth)acrylate ester compound is preferably 1 to 3 per one molecule, and more preferably 1 or 2 per one molecule.

Examples of the compound containing one or more kind selected from an alkylene skeleton and a phenylene skeleton and having an isocyanate group include methylene diisocyanate (abbr.: MDI), hexamethylene diisocyanate (abbr.: HDI), isophorone diisocyanate (abbr.: IPDI), trimethylhexamethylene diisocyanate (abbr.: TMHMDI), tricyclodecane diisocyanate (abbr.: TCDDI), adamantane diisocyanate (abbr.: ADI), tolylene diisocyanate (abbr.: TDI), xylylene diisocyanate (abbr.: XDI), and diphenylmethane diisocyanate (abbr.: DPMDI). One kind of these compounds may be used alone, or two or more kinds thereof may be used in combination. Among these, HDI, IPDI, TMHMDI, and TCDDI are preferred, and IPDI and TMHMDI are more preferred, from the standpoint of the availability and the prevention of yellowing.

Examples of the (meth)acrylate compound having a hydroxy group include a hydroxy (meth)acrylate compound, such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis(4-(3-(meth)acryloyloxy-2-hydroxypropoxy)phenyl)propane, 1,2-bis(3-(meth)acryloyloxy-2-hydroxypropoxy)ethane, pentaerythritol tri(meth)acrylate, and dipentaerythritol tri- or tetra(meth)acrylate. One kind of these compounds may be used alone, or two or more kinds thereof may be used in combination. Among these, 2-hydroxyethyl (meth)acrylate, 2-hydroxypropyl (meth)acrylate, and 2-hydroxy-3-acryloyloxypropyl (meth)acrylate are preferred, and 2-hydroxyethyl (meth)acrylate is more preferred, from the standpoint of facilitating the exhibition of the excellent adhesiveness.

The addition reaction of the compound containing one or more kind selected from an alkylene skeleton and a phenylene skeleton and containing an isocyanate group and the (meth)acrylate compound having a hydroxy group can be performed according to the ordinary method with no particular limitation.

Examples of the urethanated (meth)acrylate ester compound that does not contain a polymer structure include 2,2,4-trimethylhexamethylenebis(2-carbamoyloxyethyl) dimethacrylate (abbr.: UDMA), 2,4-tolylenebis(2-carbamoyloxyethyl) dimethacrylate, bishydroxyethyl methacrylate-isophorone diurethane, 2,4-tolylenebis(2-carbamoyloxyethyl) dimethacrylate, N,N'-(2,2,4-trimethylhexamethylene)bis[2-(aminocarboxy)propan-1,3-diol] tetramethacrylate, hexamethylenebis(2-carbamoyloxy-3-phenoxypropyl) diacrylate, and 2,4-tolylenebis(2-carbamoyloxyethyl) hexaacrylate. One kind of these compounds may be used alone, or two or more kinds thereof may be used in combination. Among these, 2,2,4-trimethylhexamethylenebis(2-carbamoyloxyethyl) dimethacrylate and N,N'-(2,2,4-trimethylhexamethylene)bis[2-(aminocarboxy)propan-1,3-diol] tetramethacrylate are preferred, and 2,2,4-trimethylhexamethylenebis(2-carbamoyloxyethyl) dimethacrylate is more preferred, from the standpoint of enhancing the adhesiveness.

In the case where the component (A) contains the urethanated (meth)acrylate ester compound, which is the polyfunctional monomer (a-1), the content of the urethanated (meth)acrylate ester compound in the component (A) is preferably 10 to 100% by mass based on 100% by mass of the component (A), and is more preferably 30 to 95% by mass, further preferably 50 to 90% by mass, and still further preferably 60 to 80% by mass, from the standpoint of achieving the further excellent adhesiveness.

In one embodiment of the present invention, the content of the urethanated (meth)acrylate ester compound is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the polyfunctional monomers (a-1).

### {Aromatic Compound based (Meth)acrylate Ester Compound containing No Urethane Bond}

Examples of the aromatic compound based (meth)acrylate ester compound containing no urethane bond include 2,2-bis((meth)acryloyloxyphenyl)propane, 2,2-bis(4-(3-acryloyloxy)-2-hydroxypropoxyphenyl)propane, 2,2-bis(4-(3-methacryloyloxy-2-hydroxypropoxy)phenyl)propane (abbr.: Bis-GMA), 2,2-bis(4-(2-(3-acryloyloxy-2-hydroxypropoxy)propyl)phenyl)propane, 2,2-bis(4-(2-(3-methacryloyloxy-2-hydroxypropoxy)propyl)phenyl)propane, 2,2-bis(4-(meth)acryloyloxyethoxyphenyl)propane, 2,2-bis(4-(meth)acyrloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl) pyromellitate. One kind of these compounds may be used alone, or two or more kinds thereof may be used in combination. Among these, a compound containing a hydroxy group is preferred, and specifically 2,2-bis(4-(3-acryloyloxy)-2-hydroxypropoxyphenyl)propane, 2,2-bis(4-(3-methacryloyloxy-2-hydroxypropoxy)phenyl)propane, 2,2-bis(4-(2-(3-acryloyloxy-2-hydroxypropoxy)propyl)phenyl)propane, 2,2-bis(4-(2-(3-methacryloyloxy-2-hydroxypropoxy)propyl)phenyl)propane are preferred, from the standpoint of achieving the excellent adhesiveness. Among these, 2,2-bis(4-(3-methacryloyloxy-2-hydroxypropoxy)phenyl)propane is more preferred.

In the case where the component (A) contains the aromatic compound based (meth)acrylate ester compound containing no urethane bond, which is the polyfunctional monomer (a-1), the content of the aromatic compound based (meth)acrylate ester compound containing no urethane bond in the component (A) is preferably 5 to 95% by mass based on 100% by mass of the component (A), and is more preferably 10 to 90% by mass, further preferably 20 to 85% by mass, still further preferably 30 to 80% by mass, and still more further preferably 50 to 70% by mass, from the standpoint of achieving the further excellent adhesiveness.

In one embodiment of the present invention, the content of the aromatic compound based (meth)acrylate ester compound containing no urethane bond is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the polyfunctional monomer (a-1).

### {Alicyclic (Meth)acrylate Ester Compound)

Examples of the alicyclic (meth)acrylate ester compound include tricyclodecanedimethanol di(meth)acrylate, cyclohexanediol di(meth)acrylate, and adamantanediol di(meth)acrylate. Among these, tricyclodecanedimethanol di(meth)acrylate is preferred.

In the case where the component (A) contains the alicyclic (meth)acrylate ester compound, which is the polyfunctional monomer (a-1), the content of the alicyclic (meth)acrylate ester compound in the component (A) is preferably 30 to 100% by mass based on 100% by mass of the component (A), and is more preferably 50 to 98% by mass, and further preferably 70 to 95% by mass, from the standpoint of achieving the further excellent strength of the denture.

In one embodiment of the present invention, the content of the alicyclic (meth)acrylate ester compound is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the polyfunctional monomer (a-1).

### {Additional Polyfunctional (Meth)acrylate Ester based Polymerizable Monomer}

Examples of a polyfunctional (meth)acrylate ester based polymerizable monomer other than the urethanated (meth)acrylate ester compound, the aromatic compound based (meth)acrylate ester compound containing no urethane bond, and the alicyclic (meth)acrylate ester compound, i.e., an additional polyfunctional (meth)acrylate ester based polymerizable monomer (which may be hereinafter referred to as an "additional polyfunctional polymerizable monomer"), include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, and dipentaerythritol penta(meth)acrylate. Among these, glycerol di(meth)acrylate, 1,2-bis(3-methacryloyloxy-2-hydroxypropoxy)ethane, and triethylene glycol di(meth)acrylate are preferred, and triethylene glycol di(meth)acrylate is more preferred, from the standpoint of achieving the further excellent adhesiveness. One kind of these compounds may be used alone, or two or more kinds thereof may be used in combination.

In the case where the component (A) contains the additional polyfunctional (meth)acrylate ester based polymerizable monomer, the content of the additional polyfunctional (meth)acrylate ester based polymerizable monomer in the component (A) is preferably 1 to 50% by mass, more preferably 5 to 40% by mass, and further preferably 8 to 35% by mass, based on 100% by mass of the component (A).

In one embodiment of the present invention, in the case where the component (A) contains the additional polyfunctional (meth)acrylate ester based polymerizable monomer, the content of the additional polyfunctional (meth)acrylate ester based polymerizable monomer is preferably 1 to 50% by mass, more preferably 1 to 40% by mass, further preferably 1 to 35% by mass, and still further preferably 1 to 30% by mass, based on 100% by mass in total of the polyfunctional monomer (a-1).

In the case where the component (A) contains the polyfunctional monomer (a-1), the total content of the polyfunctional monomer (a-1) in the component (A) is preferably 10 to 100% by mass based on 100% by mass of the component (A), and is more preferably 50 to 100% by mass, further preferably 50 to 95% by mass, still further preferably 55 to 85% by mass, and still more further preferably 60 to 80% by mass, from the standpoint of achieving the further excellent adhesiveness.

### {Monofunctional (Meth)acrylic based Polymerizable Monomer (a-2)}

As described above, the component (A) preferably contains the monofunctional monomer (a-2). The monofunctional monomer (a-2) lowers the viscosity of the resin composition (I), decreases the crosslinking density of the resulting stereolithographic article, and enhances the penetration of the adhesive composition described later, thereby enhancing the adhesiveness.

The one polymerizable group of the monofunctional monomer (a-2) means a (meth)acryloyl group.

Examples of the monofunctional monomer (a-2) include a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, a linear (meth)acrylate ester based polymerizable monomer, a cyclic (meth)acrylamide compound, and a linear (meth)acrylamide compound. From the standpoint of preventing an offensive odor in handling, a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound, which tend to have a higher boiling point, preventing an odor, are preferred, and among these, a (meth)acrylate ester based polymerizable monomer containing an aromatic ring is preferred from the standpoint of enhancing the curability of the resulting composition and the adhesiveness. In other words, the component (A) preferably contains at least one kind selected from the group consisting of a (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound, and more preferably contains a (meth)acrylate ester based polymerizable monomer containing an aromatic ring, as the monofunctional monomer (a-2) described above.

A (meth)acrylate ester based polymerizable monomer containing an aromatic ring, an alicyclic (meth)acrylate ester based polymerizable monomer, a cyclic (meth)acrylate ester based polymerizable monomer containing a nitrogen atom, and a cyclic (meth)acrylamide compound tend to have a higher boiling point and to prevent an odor as compared to a linear (meth)acrylate ester based polymerizable monomer, assuming the equivalent molecular weights therefor, and the tendency is estimated to derive from the π-electron interaction and the effect of imparting minute polarity through the fixed steric conformation.

Examples of the (meth)acrylate ester based polymerizable monomer containing an aromatic ring include o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, butoxylated o-phenylphenol (meth)acrylate, butoxylated m-phenylphenol (meth)acrylate, butoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, phenyl (meth)acrylate, 4-biphenylyl (meth)acrylate, 1-naphthyl (meth)acrylate, 2-naphthyl (meth)acrylate, anthryl (meth)acrylate, o-2-propenylphenyl (meth)acrylate, benzhydrol (meth)acrylate, cumylphenol (meth)acrylate, fluorenyl (meth)acrylate, and fluorenylmethyl (meth)acrylate.

Examples of the alicyclic (meth)acrylate ester based polymerizable monomer include 2-(1-adamantyl)propyl (meth)acrylate, 2-methyladamantyl-2-yl (meth)acrylate, 2-ethyladamantyl-2-yl (meth)acrylate, 2-n-propyladamantyl-2-yl (meth)acrylate, 2-isopropyladamantyl-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1-methylpropyl (meth)acrylate, and 1-(adamantan-1-yl)-1-ethylpropyl (meth)acrylate.

Examples of the cyclic (meth)acrylate ester based polymerizable monomer containing a nitrogen atom include pentamethylpiperidinyl (meth)acrylate, tetramethylpiperidinyl (meth)acrylate, and 4-(pyrimidin-2-yl)piperazin-1-yl (meth)acrylate.

Examples of the linear (meth)acrylate ester based polymerizable monomer include 2-ethylhexyl (meth)acrylate, decyl (meth)acrylate, undecyl (meth)acrylate, lauryl (meth)acrylate, tridecyl (meth)acrylate, tetradecyl (meth)acrylate, pentadecyl (meth)acrylate, cetyl (meth)acrylate, palmitoleyl (meth)acrylate, heptadecyl (meth)acrylate, oleyl (meth)acrylate, stearyl (meth)acrylate, isostearyl (meth)acrylate, 4-hydroxybutyl (meth)acrylate, 6-hydroxyhexyl (meth)acrylate, 10-hydroxydecyl (meth)acrylate, glycerol mono(meth)acrylate, erythritol mono(meth)acrylate, and isobornylcyclohexyl (meth)acrylate.

Examples of the cyclic (meth)acrylamide compound include N-(meth)acryloylmorpholine, N-(meth)acryloylpyrrolidine, N-(meth)acryloylpiperidine, N-(meth)acryloyl-2-methylpiperidine, and N-(meth)acryloyl-2,2,6,6-tetramethylpiperidine.

Examples of the linear (meth)acrylamide compound include N,N-dimethyl(meth)acrylamide, N,N-diethyl(meth)acrylamide, N,N-di-n-propyl(meth)acrylamide, N-isopropyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-(2-hydroxyethyl)(meth)acrylamide, and N,N,N-bis(2-hydroxyethyl)(meth)acrylamide.

Among these, the monofunctional monomer (a-2) is preferably o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, fluorenyl (meth)acrylate, fluorenylmethyl (meth)acrylate, and 4-biphenylyl (meth)acrylate, more preferably m-phenoxybenzyl (meth)acrylate, o-phenoxybenzyl (meth)acrylate, p-phenoxybenzyl (meth)acrylate, o-phenylphenol (meth)acrylate, m-phenylphenol (meth)acrylate, p-phenylphenol (meth)acrylate, ethoxylated o-phenylphenol (meth)acrylate, ethoxylated m-phenylphenol (meth)acrylate, and ethoxylated p-phenylphenol (meth)acrylate, further preferably m-phenoxybenzyl (meth)acrylate and ethoxylated o-phenylphenol (meth)acrylate, and still further preferably m-phenoxybenzyl acrylate and ethoxylated o-phenylphenol acrylate, from the standpoint of enhancing the curability of the resulting composition and enhancing the adhesiveness.

In the case where the component (A) contains the monofunctional monomer (a-2), the total content of the monofunctional monomer (a-2) in the component (A) is preferably 1 to 60% by mass based on 100% by mass of the component (A), and is preferably 5 to 50% by mass, more preferably 10 to 50% by mass, further preferably 15 to 45% by mass, and still further preferably 20 to 40% by mass from the standpoint of achieving the further excellent adhesiveness.

In one embodiment of the present invention, the content of the (meth)acrylate ester based polymerizable monomer containing an aromatic ring is preferably 50 to 100% by mass, more preferably 60 to 100% by mass, further preferably 70 to 100% by mass, still further preferably 80 to 100% by mass, still more further preferably 90 to 100% by mass, and even further preferably 95 to 100% by mass, based on 100% by mass in total of the monofunctional monomer (a-2).

In one embodiment of the present invention, the (meth)acrylic based polymerizable monomer contained in the component (A) may be constituted substantially only by the polyfunctional monomer (a-1), or the polyfunctional monomer (a-1) and the monofunctional monomer (a-2). The fact that the (meth)acrylic based polymerizable monomer is constituted substantially only by the polyfunctional monomer (a-1), or the polyfunctional monomer (a-1) and the monofunctional monomer (a-2) means the total content of the additional (meth)acrylic based polymerizable monomer other than the polyfunctional monomer (a-1), or the polyfunctional monomer (a-1) and the monofunctional monomer (a-2) is less than 10.0% by mass, preferably less than 5.0% by mass, more preferably less than 1.0% by mass, further preferably less than 0.1% by mass, and particularly preferably less than 0.01% by mass, based on 100% by mass as the total mass of the component (A).

In one embodiment of the present invention, the total content of the polyfunctional monomer (a-1) and the monofunctional monomer (a-2) in the component (A) is preferably 80 to 100% by mass, more preferably 90 to 100% by mass, further preferably 95 to 100% by mass, still further preferably 99 to 100% by mass, still more further preferably 99.9 to 100% by mass, even further preferably 99.99 to 100% by mass, based on 100% by mass of the component (A).

The content of the component (A) in the composition (I) is preferably 40% by mass or more, more preferably 45% by mass or more, further preferably 50% by mass or more, still further preferably 60% by mass or more, still more further preferably 80% by mass or more, and even further preferably 90% by mass or more, and is preferably 99.99% by mass or less, more preferably 99.5% by mass or less, further preferably 99% by mass or less, and still further preferably 98% by mass or less, based on 100% by mass of the composition (I), from the standpoint of achieving the further excellent adhesiveness. As described above, the lower limit values and the upper limit values described in a stepwise manner can be independently combined. For example, in one embodiment of the present invention, the content of the component (A) in the composition (I) is preferably 40 to 99.99% by mass, more preferably 45 to 99.99% by mass, further preferably 50 to 99.5% by mass, still further preferably 60 to 99.5% by mass, still more further preferably 80 to 99% by mass, and even further preferably 90 to 98% by mass, based on 100% by mass of the composition (I).

### <Photopolymerization Initiator>

The photopolymerization initiator may be selected from polymerization initiators that have been used in the ordinary industries, and among these, a photopolymerization initiator used in the dental field is preferred.

Examples of the photopolymerization initiator include a (bis)acylphosphine oxide compound, a thioxanthone compound or a quaternary ammonium salt of a thioxanthone compound, a ketal compound, an α-diketone compound, a coumarin compound, an anthraquinone compound, a benzoin alkyl ether compound, and an α-aminoketone based compound. One kind of the photopolymerization initiator may be used alone, or two or more kinds thereof may be used in combination.

Among these photopolymerization initiators, at least one kind selected from the group consisting of a (bis)acylphosphine oxide compound and an α-diketone compound is preferably used. With the use thereof, the composition that is excellent in photocurability in the ultraviolet region and the visible light region, and shows the sufficient photocurability by using any of light sources including a laser, a halogen lamp, a light emitting diode (LED), and a xenon lamp.

In the (bis)acylphosphine oxide compound, examples of the acylphosphine oxide compound include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyldi-(2,6-dimethylphenyl) phosphonate, 2,4,6-trimethylbenzoylphenylphosphine oxide sodium salt, 2,4,6-trimethylbenzoylphenylphosphine oxide potassium salt, and 2,4,6-trimethylbenzoyldiphenylphosphine oxide ammonium salt.

In the (bis)acylphosphine oxide compound, examples of the bisacylphosphine oxide compound include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide. Examples thereof also include the compounds described in JP2000-159621A.

Among the (bis)acylphosphine oxide compounds, at least one kind selected from the group consisting of 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt is preferably used as the photopolymerization initiator.

Examples of the α-diketone compound include diacetyl, benzyl, camphorquinone, 2,3-pentadione, 2,3-octadione, 9,10-phenanthrenequinone, 4,4'-oxybenzyl, and acenaphthenequinone. Among these α-diketone compounds, camphorquinone is preferred in the case where a light source in the visible light region is used.

While the content of the photopolymerization initiator in the composition (I) is not particularly limited, the photopolymerization initiator is preferably 0.01 to 20 parts by mass per 100 parts by mass of the component (A) from the standpoint of the curability of the resulting composition and the like. In the case where the content of the photopolymerization initiator is 0.01 part by mass or more, the polymerization can be allowed to proceed sufficiently. From this standpoint, the content of the photopolymerization initiator in the composition (I) is more preferably 0.05 part by mass or more, further preferably 0.1 part by mass or more, still further preferably 0.5 part by mass or more, and still more further preferably 1.0 part by mass or more, per 100 parts by mass of the component (A). In the case where the content of the photopolymerization initiator is 20 parts by mass or less, the photopolymerization initiator can be prevented from being deposited from the composition even in the case where the solubility thereof is low. From this standpoint, the content of the photopolymerization initiator in the composition (I) is more preferably 15 parts by mass or less, more preferably 10 parts by mass or less, and further preferably 5.0 part by mass or less, per 100 parts by mass of the component (A). As described above, the lower limit values and the upper limit values described in a stepwise manner can be independently combined. For example, in one embodiment of the present invention, the content of the photopolymerization initiator in the composition (I) is preferably 0.01 to 20 parts by mass, more preferably 0.05 to 15 parts by mass, further preferably 0.1 to 10 parts by mass, still further preferably 0.5 to 5.0 parts by mass, and still more further preferably 1.0 to 5.0 parts by mass, per 100 parts by mass of the component (A).

### <Additional Components>

The composition (I) may contain a component other than the component (A) and the photopolymerization initiator depending on necessity in such a range that does not impair the spirit of the present invention. The composition (I) can be produced according to a known method.

### (Polymerization Accelerator)

The composition (I) may contain a polymerization accelerator in a range that does not impair the spirit of the present invention, for the purpose of enhancing the photocuring capability. Examples of the polymerization accelerator include ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. Among these, at least one selected from the group consisting of ethyl 4-(N,N-dimethylamino)benzoate, n-butoxyethyl 4-(N,N-dimethylamino)benzoate, and 4-(N,N-dimethylamino)benzophenone is preferably used from the standpoint of imparting the excellent curability to the composition (I). One kind of the polymerization initiator may be used alone, or two or more kinds thereof may be used in combination.

### (Filler)

The composition (I) may contain a filler from the standpoint of regulating the viscosity of the composition, imparting the strength and the antibacterial activity to the denture base, and the like. Examples of the filler include an organic filler, an inorganic filler, and an organic-inorganic composite filler. One kind of the filler may be used alone, or two or more kinds thereof may be used in combination.

Examples of the material of the organic filler include polymethyl methacrylate, polyethyl methacrylate, a methyl methacrylate-ethyl methacrylate copolymer, crosslinked polymethyl methacrylate, crosslinked polyethyl methacrylate, polyester, polyamide, polycarbonate, polyphenylene ether, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. One kind of these materials may be used alone, or two or more kinds thereof may be used in combination. The shape of the organic filler is not particularly limited, and the particle diameter of the filler can be appropriately selected in use.

Examples of the material of the inorganic filler include quartz, silica, alumina, silica-titania, silica-titania-barium oxide, silica-zirconia, silica-alumina, lanthanum glass, borosilicate glass, soda glass, barium glass, strontium glass, glass ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. One kind of these materials may be used alone, or two or more kinds thereof may be used in combination. The shape of the organic filler is not particularly limited, and an irregular shape filler or a spherical filler can be appropriately selected in use.

Examples of the organic-inorganic composite filler include an organic-inorganic composite filler obtained by dispersing an inorganic based filler in an organic based filler, and an organic-inorganic composite filler obtained by coating an inorganic based filler with various kinds of a polymerizable monomer.

In the case where the composition (I) contains the filler, for example, the content of the filler in the composition (I) is preferably 0.1 part by mass or more, more preferably 0.5 part by mass or more, and further preferably 1 part by mass or more, per 100 parts by mass of the component (A), from the standpoint of regulating the viscosity of the composition, imparting the strength to the denture base, and the like. The content of the filler in the composition (I) is preferably 20 parts by mass or less, more preferably 10 parts by mass or less, and further preferably 5 parts by mass or less, per 100 parts by mass of the component (A), from the standpoint of suppressing the influence thereof on formability due to the viscosity increase of the composition (I). As described above, the lower limit values and the upper limit values described in a stepwise manner can be independently combined. For example, in one embodiment of the present invention, in the case where the composition (I) contains the filler, the content of the filler in the composition (I) is preferably 0.1 to 20 parts by mass, more preferably 0.5 to 10 parts by mass, and further preferably 1 to 5 parts by mass, per 100 parts by mass of the component (A).

### (Polymer)

The composition (I) may contain a polymer for the purpose of modifying the flexibility, the flowability, and the like, in such a range that does not impair the spirit of the present invention. For example, natural rubber, synthetic polyisoprene rubber, liquid polyisoprene rubber or a hydrogenated product thereof, polybutadiene rubber, liquid polybutadiene rubber or a hydrogenated product thereof, styrene-butadiene rubber, chloroprene rubber, ethylenepropylene rubber, acrylic rubber, isoprene-isobutylene rubber, acrylonitrile-butadiene rubber, or a styrene based elastomer may be added. Specific examples of other polymers that can be added include a polystyrene-polyisoprene-polystyrene block copolymer, a polystyrenepolybutadiene-polystyrene block copolymer, a poly(α-methylstyrene)-polybutadiene-poly(α-methylstyrene) block copolymer, a poly(p-methylstyrene)-polybutadiene-poly(p-methylstyrene) block copolymer, and hydrogenated products thereof. One kind of the polymer may be used alone, or two or more kinds thereof may be used in combination.

### (Softener)

The composition (I) may contain a softener depending on necessity. Examples of the softener include a petroleum based softener, such as paraffin based, naphthene based, and aromatic based process oils, and a vegetable oil based softener, such as paraffin, peanut oil, and rosin. One kind of the softener may be used alone, or two or more kinds thereof may be used in combination. In the case where the softener is used, the content of the softener in the composition (I) is not particularly limited unless the spirit of the present invention is impaired, and is generally preferably 1 to 30 parts by mass, and more preferably 5 to 20 parts by mass, per 100 parts by mass of the component (A).

### (Stabilizer)

The composition (I) may contain a known stabilizer for the purpose of preventing the deterioration or regulating the photocurability. Examples of the stabilizer include a polymerization inhibitor, an ultraviolet ray absorbent, and an antioxidant.

Examples of the polymerization inhibitor include hydroquinone, hydroquinone monomethyl ether, dibutylhydroquinone, dibutylhydroquinone monomethyl ether, 4-t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 5.0 parts by mass, more preferably 0.01 to 3.0 parts by mass, and further preferably 0.1 to 2.0 parts by mass, per 100 parts by mass of the component (A).

### (Colorant)

The composition (I) may contain a colorant in such a range that does not impair the spirit of the present invention, for example, in the case where the color of the denture base is to be regulated to the gingival color or the like. The colorant used may be, for example, a pigment or a dye. The pigment can be roughly classified into an inorganic pigment and an organic pigment, which may be mixed in use.

Examples of the inorganic pigment include an oxide, a hydroxide, a sulfide, a chromate salt, a silicate salt, a sulfate salt, a carbonate salt, a ferrocyanic compound, a phosphate salt, and carbon, and among these, an oxide is preferably used. Specific examples of the oxide include red iron oxide, yellow iron oxide, black iron oxide, titanium oxide (TiO₂), alumina, and zirconia. The red iron oxide is colcothar or hematite and is iron oxide shown by the chemical formula Fe₂O₃, yellow iron oxide is goethite and is iron oxide shown by the chemical formula FeOOH, and black iron oxide is magnetite and is iron oxide shown by the chemical formula Fe₃O₄.

Examples of the organic pigment include an azo based pigment, a phthalocyanine pigment, a condensed polycyclic pigment, a nitro based pigment, a nitroso based pigment, and a fluorescent pigment, and among these, an azo based pigment and a phthalocyanine pigment are preferably used. Specific examples of the azo based pigment include Novoperm Yellow 4G, Chromophthal Yellow 3G, Chromophthal Yellow 3RLP, Novoperm Red BN, Chromophthal Scarlet RN, Chromophthal Blue 4GNP, and Blue No.2 aluminum lake. Specific examples of the phthalocyanine pigment include Phthalocyanine Blue and Phthalocyanine Green.

For example, in the case where the color of the composition (I) is regulated to the color of the gingival color, preferred examples of the pigment that is mainly used include red iron oxide.

In the case where the composition (I) contains the colorant, the total content of the colorant in the composition (I) is not particularly limited, and for example, is preferably 0.0005 to 5.0 parts by mass per 100 parts by mass of the component (A). In the case where the colorant is a pigment, the preferred ranges of the total content thereof are also the same.

### (Additional Additive)

The composition (I) may contain a known additional additive depending on necessity, for example, for regulating the paste properties, in addition to the components described above in such a range that does not impair the spirit of the present invention. Examples of the additional additive include a thickener, a prepolymer (oligomer), and an aryl compound (such as an aryl iodonium salt). One kind of the additional additive may be used alone, or two or more kinds thereof may be used in combination.

The method of providing the denture base that is a stereolithographic article of the composition (I) is not particularly limited, as far as being stereolithography, and known method and apparatus can be used. The stereolithography can be performed by using known method and apparatus. For example, a method using a CAD/CAM system or the like, such as a method using the stereolithographic 3D printer described above, can be used.

The denture base that is a stereolithographic article of the composition (I) may be a denture base containing the stereolithographic article of the composition (I) as a part thereof, or may be a denture base containing the stereolithographic article of the composition (I) as the whole thereof, and it is that at least a part of the part thereof to be brought into contact with the adhesive composition described later is the stereolithographic article of the composition (I), and it is preferred that the whole of the part thereof to be brought into contact with the adhesive composition is the stereolithographic article of the composition (I).

### [Acrylic based Resin (II)]

The acrylic based resin (II) is a component constituting the prosthetic tooth, and is a polymer of a (meth)acrylic based polymerizable monomer, and preferably a polymer of a (meth)acrylate ester based polymerizable monomer.

Examples of the (meth)acrylic based polymerizable monomer that can be used in the acrylic based resin (II) include the (meth)acrylate ester based polymerizable compound described for the curable composition (I) above, and examples thereof also include methyl methacrylate and ethyl methacrylate.

The acrylic based resin (II) may further contain a filler for imparting the strength and the antibacterial activity to the prosthetic tooth. Examples of the filler include the organic fillers, the inorganic fillers, and the organic-inorganic composite fillers described for the curable composition (I) above. One kind of the filler may be used alone, or two or more kinds thereof may be used in combination.

The composition (II) may further contain, for example, a colorant from the standpoint of regulating the color of the prosthetic tooth to the tooth crown color or the like. Examples of the colorant include the colorants described for the composition (I) above. One kind of the colorant may be used alone, or two or more kinds thereof may be used in combination.

The composition (II) may contain various additives that have been used in a prosthetic tooth in such a range that does not impair the spirit of the present invention.

The prosthetic tooth is a cured product of the acrylic based resin (II), and the method of providing the prosthetic tooth from the acrylic based resin (II) is not particularly limited, and may be any of known method of and apparatus, examples of which include a method of molding the acrylic based resin (II) into the prosthetic tooth shape and subjecting to photopolymerization or thermal polymerization, a method of cutting a block or disk material formed by molding and polymerizing, and a method of inj ection-molding a thermoplastic resin formed by polymerizing a material containing PMMA as a major material.

The prosthetic tooth containing a cured product of the acrylic based resin (II) may be a prosthetic tooth containing the cured product of the acrylic based resin (II) as a part thereof, or may be a prosthetic tooth containing the cured product of the acrylic based resin (II) as the whole thereof, and it is that at least a part of the part thereof to be brought into contact with the adhesive composition described later is the cured product of the acrylic based resin (II), and it is preferred that the whole of the part thereof to be brought into contact with the adhesive composition described later is the cured product of the acrylic based resin (II).

The prosthetic tooth that is a cured product of the acrylic based resin (II) may be a commercially available product, and examples thereof include a resin tooth "Veracia (registered trademark)" series, available from SHOFU INC., and a resin tooth "Surpass (registered trademark)" series, available from GC Corporation.

### [Adhesive Composition]

The adhesive composition contains a composition (III) containing a (meth)acrylic based polymerizable monomer component (C) containing the same (meth)acrylic based polymerizable monomer (m) as contained in the curable composition (I) (which may be hereinafter referred simply to as a "composition (III)").

As described above, the adhesive composition is used as an adhesive for adhering the denture base that is a stereolithographic article of the composition (I) and the prosthetic tooth containing the acrylic based resin (II). According to the mechanism described above, it is estimated that the use of the composition (III) as an adhesive achieves firm adhesiveness to the denture base that is a stereolithographic article of the composition (I). It is also estimated that the composition (III) has affinity with the acrylic based resin (II), and thereby can further firmly adhere the denture base and the prosthetic tooth.

### <Composition (III)>

The composition (III) contains a (meth)acrylic based polymerizable monomer component (C) (which may be hereinafter referred simply to as a "component (C)") containing the same (meth)acrylic based polymerizable monomer (m) as contained in the curable composition (I).

The composition (III) preferably has curability, and more preferably has photocurability.

### ((Meth)acrylic based Polymerizable Monomer Component (C))

The (meth)acrylic based polymerizable monomer component (C) is a component that is constituted by one kind of a (meth)acrylic based polymerizable monomer alone or two or more kinds thereof, and contains the same monomer (m) as contained in the composition (I).

As described above, the component (C) contains the same (meth)acrylic based polymerizable monomer as the monomer (m), which is the (meth)acrylic based polymerizable monomer contained as the major monomer in the (meth)acrylic based polymerizable monomers constituting the component (A), and thereby excellent adhesiveness can be obtained.

The "same (meth)acrylic based polymerizable monomer as the monomer (m)" herein means the sameness as the monomer (m) in terms of specific compound. For example, in the case where the monomer (m) in the component (A) is UDMA, the monomer (m) that the component (C) necessarily contains is UDMA in question.

Therefore, for example, in the case where the monomer (m) in the component (A) is UDMA, which is the polyfunctional monomer (a-1), the fact that the component (C) contains Bis-GMA, which is in the same category as the polyfunctional monomer (a-1), does not satisfy the requirement that the "same (meth)acrylic based polymerizable monomer as the monomer (m)" is contained.

In the case where multiple kinds of the monomers (m) as the major monomers exist in the component (A), for example, in the case where two kinds of the (meth)acrylic based polymerizable monomers are contained each in an amount of 50% by mass in the component (A), it suffices that the component (C) contains any one of the monomers (m) in the component (A), and it is not necessary that all the monomers (m) in the component (A) are contained.

As described above, the content of the monomer (m) in the component (C) is preferably 20% by mass or more, more preferably 30% by mass or more, further preferably 40% by mass or more, still further preferably 50% by mass or more, still more further preferably 55% by mass or more, even further preferably 60% by mass or more, and even still further preferably 65% by mass or more, and is for example, 100% by mass or less, preferably 98% by mass or less, more preferably 95% by mass or less, further preferably 90% by mass or less, still further preferably 85% by mass or less, and still more further preferably 80% by mass or less, based on 100% by mass of the component (C), from the standpoint achieving the further excellent adhesiveness. As described above, the lower limit values and the upper limit values described in a stepwise manner can be independently combined. For example, in one embodiment of the present invention, the content of the monomer (m) in the component (C) is preferably 20 to 100% by mass, more preferably 30 to 100% by mass, further preferably 40 to 98% by mass, still further preferably 50 to 95% by mass, still more further preferably 55 to 90% by mass, even further preferably 60 to 85% by mass, and even still further preferably 65 to 80% by mass, based on 100% by mass of the component (C).

The (meth)acrylic based polymerizable monomer used for constituting the component (C) may be the similar (meth)acrylic based polymerizable monomer as described above for the component (A), and the preferred kinds of the (meth)acrylic based polymerizable monomer therefor are also the same as described for the component (A). The content of the (meth)acrylic based polymerizable monomers that the component (C) may contain and the preferred ranges thereof are also the same as described for the component (A) except that the description "based on 100% by mass of the component (A)" is changed to "based on 100% by mass of the component (C)", as far as satisfying the requirement to contain the monomer (m). The reasons for the preferred embodiments and the preferred ranges thereof are also the same as described above unless otherwise indicated.

As described above, the component (C) is a component that is constituted only by the monomer (m) alone, or by the monomer (m) and the monomer (o). The component (C) may contain multiple kinds of the monomer (o). Therefore, the total content of the monomer (m) and the monomer (o) in the component (C) is 100% by mass.

In one embodiment of the present invention, it is preferred that not only the component (C) contains the same monomer (m) as the component (A), but also the monomer (o) contained therein is the same (meth)acrylic based polymerizable monomer as the monomer (o) contained in the component (A). The "same (meth)acrylic based polymerizable monomer as the monomer (o)" herein is the same as in the description for the "same (meth)acrylic based polymerizable monomer as the monomer (m)". In the case where the component (A) contains multiple compounds as the monomer (o), the component (C) preferably contains all the multiple compounds as the monomer (o).

In one embodiment of the present invention, furthermore, the absolute value of the difference between the content of the monomer (m) in the component (C) and the content of the monomer (m) in the component (A) is preferably 10% by mass or less, more preferably 5% by mass or less, further preferably 3% by mass or less, and still further preferably 1% by mass or less. In other words, in one embodiment of the present invention, the absolute value of the difference between the content of the monomer (m) in the component (C) and the content of the monomer (m) in the component (A) is preferably 0 to 10% by mass, more preferably 0 to 5% by mass, further preferably 0 to 3% by mass, and still further preferably 0 to 1% by mass.

Similarly, the absolute value of the difference between the content of the monomer (o) in the component (C) and the content of the monomer (o) in the component (A) is preferably 10% by mass or less, more preferably 5% by mass or less, further preferably 3% by mass or less, and still further preferably 1% by mass or less. In other words, in one embodiment of the present invention, the absolute value of the difference between the content of the monomer (o) in the component (C) and the content of the monomer (o) in the component (C) is preferably 0 to 10% by mass, more preferably 0 to 5% by mass, further preferably 0 to 3% by mass, and still further preferably 0 to 1% by mass. In the case where the component (A) contains multiple compounds as the monomer (o), and the component (C) also contains the same multiple compounds as in the component (A) as the monomer (o), the absolute values of the differences between the content in the component (A) and the content in the component (C) of the corresponding compounds each are independently preferably 10% by mass or less, more preferably 5% by mass or less, further preferably 3% by mass or less, and still further preferably 1% by mass or less. In other words, in one embodiment of the present invention, in the case where the component (A) contains multiple compounds as the monomer (o), and the component (C) also contains the same multiple compounds as in the component (A) as the monomer (o), the absolute values of the differences between the content in the component (A) and the content in the component (C) of the corresponding compounds each are independently preferably 0 to 10% by mass, more preferably 0 to 5% by mass, further preferably 0 to 3% by mass, and still further preferably 0 to 1% by mass.

However, as described above, the total content of the monomer (m) and the monomer (o) in the component (C) is 100% by mass.

In one embodiment of the present invention, the component (C) preferably contains the (meth)acrylic based polymerizable monomer component (A) in an amount of 70% by mass or more, more preferably 80% by mass or more, and further preferably 90% by mass or more, and in an amount of 100% by mass or less, based on 100% by mass of the (meth)acrylic based polymerizable monomer component (C), from the standpoint of achieving the further excellent adhesiveness. In other words, in one embodiment of the present invention, the component (C) preferably contains the (meth)acrylic based polymerizable monomer component (A) in an amount of 70 to 100% by mass, more preferably 80 to 100% by mass, and further preferably 90 to 100% by mass, based on 100% by mass of the (meth)acrylic based polymerizable monomer component (C). In one embodiment of the present invention, the component (C) still further preferably contains the (meth)acrylic based polymerizable monomer component (A) in an amount of 100% by mass based on 100% by mass of the (meth)acrylic based polymerizable monomer component (C). In other words, it is still further preferred to use the component (A) as the component (C).

In one embodiment of the present invention, the composition of the component (C) (i.e., the combination of the (meth)acrylic based polymerizable monomers and the content ratio thereof (mass ratio)) is even further preferably the same as the composition of the component (A) (i.e., the combination of the (meth)acrylic based polymerizable monomers and the content ratio thereof (mass ratio)).

The content of the component (C) in the composition (III) is preferably 40% by mass or more, more preferably 45% by mass or more, further preferably 50% by mass or more, still further preferably 60% by mass or more, still more further preferably 80% by mass or more, even further preferably 90% by mass or more, and even still further preferably 95% by mass or more, and is 100% by mass or less, preferably 99.99% by mass or less, more preferably 99.5% by mass or less, further preferably 99% by mass or less, and still further preferably 98% by mass or less, based on 100% by mass of the composition (III), from the standpoint of achieving the further excellent adhesiveness. As described above, the lower limit values and the upper limit values described in a stepwise manner can be independently combined. For example, in one embodiment of the present invention, the content of the component (C) in the composition (III) is preferably 40 to 100% by mass, more preferably 45 to 99.99% by mass, further preferably 50 to 99.99% by mass, still further preferably 60 to 99.5% by mass, still more further preferably 80 to 99% by mass, even further preferably 90 to 99% by mass, and even still further preferably 95 to 98% by mass, based on 100% by mass of the composition (III).

### (Additional Components that Composition (III) may contain)

The composition (III) may contain a component other than the component (C) depending on necessity in such a range that does not impair the spirit of the present invention. The composition (III) can be produced according to a known method.

The composition (III) preferably contains a photopolymerization initiator from the standpoint of achieving the further excellent adhesiveness.

Examples of the photopolymerization initiator that the composition (III) can use include the same photopolymerization initiators as described for the photopolymerization initiator contained in the composition (I), and the kinds of the preferred photopolymerization initiators therefor are also the same as the description for the composition (I).

In the case where the composition (III) contains a photopolymerization initiator, the preferred ranges of the content thereof are also the same as described for the photopolymerization initiator contained in the composition (I) except that the description "composition (I)" is changed to "composition (III)", and the description "per 100 parts by mass of the component (A)" is changed to "per 100 parts by mass of the component (C)".

The composition (III) may contain a polymerization accelerator in a range that does not impair the spirit of the present invention. Examples of the polymerization accelerator that the composition (III) can use include the same polymerization accelerators as described for the polymerization accelerator that the composition (I) may contain. One kind of the polymerization accelerator may be used alone, or two or more kinds thereof may be used in combination.

The composition (III) may contain a filler in a range that does not impair the spirit of the present invention for regulating the viscosity of the composition.

Examples of the filler that the composition (III) can use include the organic fillers, the inorganic fillers, and the organic-inorganic composite fillers described for the curable composition (I) above. One kind of the filler may be used alone, or two or more kinds thereof may be used in combination.

In the case where the composition (III) contains the filler, for example, the content of the filler in the composition (III) is preferably 0.1 part by mass or more, more preferably 0.3 part by mass or more, and further preferably 0.5 part by mass or more, per 100 parts by mass of the component (C), from the standpoint of regulating the viscosity of the composition. The content of the filler in the composition (III) is preferably 10 parts by mass or less, more preferably 5 parts by mass or less, and further preferably 3 parts by mass or less, per 100 parts by mass of the component (C), from the standpoint of suppressing the influence thereof on the coatability due to the viscosity increase of the adhesive composition. As described above, the lower limit values and the upper limit values described in a stepwise manner can be independently combined. For example, in one embodiment of the present invention, in the case where the composition (III) contains the filler, for example, the content of the filler in the composition (III) is preferably 0.1 to 10 parts by mass, more preferably 0.3 to 5 parts by mass, and further preferably 0.5 to 3 parts by mass, per 100 parts by mass of the component (C).

The composition (III) may contain a polymer in such a range that does not impair the spirit of the present invention. Examples of the polymer that the composition (III) may use include the same polymers as described for the polymer that the composition (I) may contain. One kind of the polymer may be used alone, or two or more kinds thereof may be used in combination.

The composition (III) may contain a known stabilizer for the purpose of preventing the deterioration or regulating the curability. Examples of the stabilizer include a polymerization inhibitor, an ultraviolet ray absorbent, and an antioxidant.

Examples of the polymerization inhibitor that the composition (III) may use include the same polymerization inhibitors as described for the polymer that the composition (I) may contain. One kind of the polymerization inhibitor may be used alone, or two or more kinds thereof may be used in combination.

In the case where the composition (III) contains the polymerization inhibitor, the content of the polymerization inhibitor is preferably 0.001 to 5.0 parts by mass, more preferably 0.01 to 3.0 parts by mass, and further preferably 0.1 to 2.0 parts by mass, per 100 parts by mass of the component (C).

The composition (III) may contain a colorant in such a range that does not impair the spirit of the present invention, for example, for further enhancing the good appearance of the resulting denture by regulating the color of the adhesive composition to be close to the denture base or the prosthetic tooth. Examples of the colorant include the colorants described for the composition (I) above. One kind of the colorant may be used alone, or two or more kinds thereof may be used in combination.

In the case where the color of the adhesive composition is regulated to the color of the denture base, for example, the gingival color, examples of the pigment that is mainly used include red iron oxide. In the case where the color of the adhesive composition is regulated to the color of the prosthetic tooth, for example, the tooth crown color, it is preferred that titanium oxide or alumina are used as a major component, and the amounts of red iron oxide, yellow iron oxide, and black iron oxide added thereto are regulated depending on the desired color. The expression "titanium oxide or alumina are used as a major component" means that the colorant that has the largest content in the colorants added to the composition is titanium oxide or alumina.

In the case where the composition (III) contains the colorant, the total content of the colorant in the composition (III) is not particularly limited, and for example, is preferably 0.0005 to 5.0 parts by mass per 100 parts by mass of the component (C). In the case where the colorant is a pigment, the preferred ranges of the total content thereof are also the same.

The composition (III) may contain a known additional additive depending on necessity, for example, for regulating the paste properties, in addition to the components described above in such a range that does not impair the spirit of the present invention. Examples of the additional additive include a thickener, a softener, a prepolymer (oligomer), and an aryl compound (such as an aryl iodonium salt). One kind of the additional additive may be used alone, or two or more kinds thereof may be used in combination.

In one embodiment of the present invention, the composition (III) preferably contains the composition (I) in an amount of 70% by mass or more, more preferably 80% by mass or more, and further preferably 90% by mass or more, and is preferably 100% by mass or less, based on 100% by mass of the composition (III), from the standpoint of achieving the further excellent adhesiveness. In other words, in one embodiment of the present invention, the composition (III) preferably contains the composition (I) in an amount of 70 to 100% by mass, more preferably 80 to 100% by mass, and further preferably 90 to 100% by mass, based on 100% by mass of the composition (III). In the embodiment of the present invention, the composition (III) still further preferably contains the composition (I) in an amount of 100% by mass based on 100% by mass of the composition (III) from the standpoint of achieving the further excellent adhesiveness. In other words, it is still further preferred to use the composition (I) as the composition (III).

### <Additional Components>

The adhesive composition may contain a component other than the composition (III) depending on necessity in such a range that does not impair the spirit of the present invention. For example, the adhesive composition may contain a polymerizable monomer, an organic solvent, and an additional additive that are not contained in the composition (III), for regulating the coatability and the penetration of the adhesive composition to the denture base and the prosthetic tooth.

Examples of the organic solvent include ethyl acetate, butyl acetate, methylene chloride, acetone, ethanol, isopropanol, and methyl methacrylate. One kind of the organic solvents may be used alone, or two or more kinds thereof may be used in combination. Among these, ethyl acetate, ethanol, and isopropanol are preferred from the standpoint of the safety and the appropriate volatility.

In the case where the adhesive composition contains the organic solvent, the content of the organic solvent is preferably 90% by mass or less, more preferably 80% by mass or less, further preferably 60% by mass or less, still further preferably 50% by mass or less, still more further preferably 40% by mass or less, even further preferably 20% by mass or less, and even still further preferably 10% by mass or less, based on 100% by mass of the adhesive composition. In the case where the adhesive composition contains the organic solvent, the lower limit of the content of the organic solvent is not particularly limited, and is for example, preferably 1% by mass or more based on 100% by mass of the adhesive composition. As described above, the lower limit values and the upper limit values described in a stepwise manner can be independently combined. For example, in one embodiment of the present invention, in the case where the adhesive composition contains the organic solvent, the content of the organic solvent is preferably 1 to 90% by mass, more preferably 1 to 80% by mass, further preferably 1 to 60% by mass, still further preferably 1 to 50% by mass, still more further preferably 1 to 40% by mass, even further preferably 1 to 20% by mass, and even still further preferably 1 to 10% by mass, based on 100% by mass of the adhesive composition.

The content of the composition (III) is preferably 10% by mass or more, more preferably 20% by mass or more, further preferably 40% by mass or more, still further preferably 50% by mass or more, still more further preferably 60% by mass or more, even further preferably 80% by mass or more, and even still further preferably 90% by mass or more, and is preferably 100% by mass or less, based on 100% by mass of the adhesive composition, from the standpoint of achieving the further excellent adhesiveness. In other words, in one embodiment of the present invention, the content of the composition (III) is preferably 10 to 100% by mass, more preferably 20 to 100% by mass, further preferably 40 to 100% by mass, still further preferably 60 to 100% by mass, still more further preferably 80 to 100% by mass, and even further preferably 90 to 100% by mass, based on 100% by mass of the adhesive composition. In one embodiment of the present invention, the adhesive composition even still further preferably contains the composition (III) in an amount of 100% by mass based on 100% by mass of the adhesive composition. In other words, it is even still further preferred to use the composition (III) as the adhesive composition.

### [Adhering Step]

The production method is not particularly limited, as far as including a step of adhering the denture base that is a stereolithographic article of the composition (I) and the prosthetic tooth containing the acrylic based resin (II) with the adhesive composition (which may be hereinafter referred simply to as an "adhering step").

Therefore, the present invention encompasses, as one embodiment, a method of adhering a denture base and a prosthetic tooth, including adhering a denture base that is a stereolithographic article of the composition (I) and a prosthetic tooth containing the acrylic based resin (II) with the adhesive composition.

In the adhering step, the denture base and the prosthetic tooth can be adhered by coating the adhesive composition on an adhesion surface of one of the denture base and the prosthetic tooth or adhesion surfaces of both of them, bonding the denture base and the prosthetic tooth to each other via the adhesive composition, and curing the adhesive composition. In the adhering step, it is preferred that the adhesion is performed by photocuring the adhesive composition.

Specifically, for example, the production method in one embodiment preferably includes the following steps (1) and (2) in this order.
Step (1): A step of coating the adhesive composition on at least one selected from the denture base and the prosthetic tooth
Step (2): A step of adhering the denture base and the prosthetic tooth via the adhesive composition under pressure

The production method more preferably includes the following step (3) after the step (2).

Step (3): A step of adhering the denture base and the prosthetic tooth by photocuring the adhesive composition

The coating method of the adhesive composition on the denture base and/or the prosthetic tooth is not particularly limited, and known method and device or apparatus can be used. For example, the method can be manually performed with a brush or the like.

The surface of the denture base to be brought into contact with the adhesive composition may be polished in advance.

Similarly, the surface of the prosthetic tooth to be brought into contact with the adhesive composition may be polished in advance.

The method of producing a denture as one embodiment of the present invention can produce a denture including a denture base and a prosthetic tooth united to each other through firm adhesion of the denture base and the prosthetic tooth.

In stereolithography, secondary polymerization is generally performed after producing a stereolithographic article, for the purpose of polymerizing the unreacted polymerizable monomer derived from the composition for forming the stereolithographic article. The method of performing the secondary polymerization is not particularly limited, and may be performed by using known method and apparatus, and a dental light irradiator, a secondary polymerization device for stereolithography, or the like can be used therefor.

In one embodiment of the present invention, as for the denture base that is a stereolithographic article and/or the prosthetic tooth that is obtained as a stereolithographic article, the adhesive composition may be coated on the denture base and/or the prosthetic tooth before the secondary polymerization of the denture base and/or the prosthetic tooth, which are then adhered to each other, and thereby higher adhesiveness can be obtained.

Accordingly, the denture base and the prosthetic tooth each encompass a material before the secondary polymerization.

In the case where the adhesive composition is coated before the secondary polymerization, and then the secondary polymerization is performed, it is considered that the unreacted polymerizable monomer in the denture base and/or the prosthetic tooth can be copolymerized with the polymerizable monomer constituting the adhesive composition, which thus facilitate the achievement of higher adhesiveness.

The method of producing a denture as one embodiment of the present invention can also achieve excellent adhesiveness even in the state where the polymerization has highly progressed through the secondary polymerization and after a long period of time has passed since the stereolithography, and can produce a denture that is sufficiently excellent in adhesiveness even after a long period of time has passed since the production of the denture base and/or the prosthetic tooth.

In adhering the denture base and the prosthetic tooth by using the adhesive composition, a known dental light irradiator (for example, "α-Light V", available from J. MORITA TOKYO MFG. CORP.), a post-cure apparatus for stereolithography (for example, "Otoflash (registered trademark) G171", available from Envision TEC, Inc.), and the like can be used. The light energy used for curing the adhesive composition is preferably an active energy light ray. In other words, it is preferred that the adhesive composition is cured by irradiating the adhesive composition with an active energy light ray, so as to adhere the denture base and the prosthetic tooth.

Therefore, the step (3) is more preferably the following step (3A).

Step (3A): A step of adhering the denture base and the prosthetic tooth by curing the adhesive composition through irradiation of the adhesive composition with an active energy light ray

The "active energy light ray" herein means an energy ray that is capable of curing a resin composition for stereolithography, such as an ultraviolet ray, an electron beam, an X-ray, a radiation, and a high frequency wave. Examples of the source of the active energy light ray include an illumination, such as a halogen lamp, a xenon lamp, a metal halide lamp, an LED, a mercury lamp, and a fluorescent lamp, and a halogen lamp and an LED are preferred among these.

### Examples

The present invention will be described more specifically with reference to examples below, but the present invention is not limited to the examples.

The components used in Examples and Comparative Examples and abbreviated names thereof will be described below.

### [(Meth)acrylic based Polymerizable Monomer]

### <Polyfunctional (Meth)acrylic based Polymerizable Monomer (a-1)>

"UDMA": 2,2,4-trimethylhexamethylenebis(2-carbamoyloxyethyl) dimethacrylate (available from Kyoeisha Chemical Co., Ltd., molecular weight: 471)
"Bis-GMA": 2,2-bis(4-(3-methacryloyloxy)-2-hydroxypropoxyphenyl)propane (available from SHIN-NAKAMURA CHEMICAL Co., Ltd.)
"TCDDMA": tricyclodecanedimethanol dimethacrylate (available from Kyoeisha Chemical Co., Ltd.)
"TEGDMA": triethylene glycol dimethacrylate (available from SHIN-NAKAMURA CHEMICAL Co., Ltd.)

### <Monofunctional (Meth)acrylic based Polymerizable Monomer (a-2)>

"POBA": m-phenoxybenzyl acrylate (available from Kyoeisha Chemical Co., Ltd.)
"EPPA": ethoxylated o-phenylphenol acrylate (available from SHIN-NAKAMURA CHEMICAL Co., Ltd.)

### [Photopolymerization Initiator]

"TPO": 2,4,6-trimethylbenzoyldiphenylphosphine oxide

### [Polymerization Inhibitor]

"BHT": 3,5-di-t-butyl-4-hydroxytoluene

### <Reference Example 1>

### [Production of Curable Composition (I)-1]

700 g of UDMA, 300 g of POBA, 30 g of TPO, and 5.0 g of BHT were placed in a 2 L brown polyethylene wide mouthed bottle, to which a mechanical agitator was inserted, and the components were agitated at 25°C for 48 hours and confirmed complete dissolution. The resulting composition was designated as a curable composition (I)-1. The composition of the curable composition (I)-1 is shown in Table 1 below.

### <Reference Example 2>

### [Production of Curable Composition (I)-2]

600 g of Bis-GMA, 400 g of POBA, 30 g of TPO, and 5.0 g of BHT were placed in a 2 L brown polyethylene wide mouthed bottle, to which a mechanical agitator was inserted, and the components were agitated at 25°C for 48 hours and confirmed complete dissolution. The resulting composition was designated as a curable composition (I)-2. The composition of the curable composition (I)-2 is shown in Table 1 below.

### <Reference Example 3>

### [Production of Curable Composition (I)-3]

900 g of TCDDMA, 100 g of EPPA, 30 g of TPO, and 5.0 g of BHT were placed in a 2 L brown polyethylene wide mouthed bottle, to which a mechanical agitator was inserted, and the components were agitated at 25°C for 48 hours and confirmed complete dissolution. The resulting composition was designated as a curable composition (I)-3. The composition of the curable composition (I)-3 is shown in Table 1 below.

### <Reference Example 4>

### [Production of Curable Composition (I)-4]

700 g of UDMA, 300 g of TEGDMA, 30 g of TPO, and 5.0 g of BHT were placed in a 2 L brown polyethylene wide mouthed bottle, to which a mechanical agitator was inserted, and the components were agitated at 25°C for 48 hours and confirmed complete dissolution. The resulting composition was designated as a curable composition (I)-4. The composition of the curable composition (I)-4 is shown in Table 1 below.

### <Reference Example 5>

### [Production of Curable Composition (I)-5]

900 g of TCDDMA, 100 g of TEGDMA, 30 g of TPO, and 5.0 g of BHT were placed in a 2 L brown polyethylene wide mouthed bottle, to which a mechanical agitator was inserted, and the components were agitated at 25°C for 48 hours and confirmed complete dissolution. The resulting composition was designated as a curable composition (I)-5. The composition of the curable composition (I)-5 is shown in Table 1 below.

### <Reference Example 6>

### [Production of Curable Composition (I)-6]

400 g of UDMA, 300 g of TEGDMA, 300g of POBA, 30 g of TPO, and 5.0 g of BHT were placed in a 2 L brown polyethylene wide mouthed bottle, to which a mechanical agitator was inserted, and the components were agitated at 25°C for 48 hours and confirmed complete dissolution. The resulting composition was designated as a curable composition (I)-6. The composition of the curable composition (I)-6 is shown in Table 1 below.

**Table 1**

| | | | Unit | Reference Example 1 | Reference Example 2 | Reference Example 3 | Reference Example 4 | Reference Example 5 | Reference Example 6 |
|---|---|---|---|---|---|---|---|---|---|
| Composition No. | | | | (I)-1 | (I)-2 | (I)-3 | (I)-4 | (I)-5 | (I)-6 |
| Component (A) (*1) | Monomer (a-1) (*2) | UDMA | g | 700 | - | - | 700 | - | 400 |
| | | Bis-GMA | g | - | 600 | - | - | - | - |
| | | TCDDMA | g | - | - | 900 | - | 900 | - |
| | | TEGDMA | g | - | - | - | 300 | 100 | 300 |
| | Monomer (a-2) (*3) | POBA | g | 300 | 400 | - | - | - | 300 |
| | | EPPA | g | - | - | 100 | - | - | - |
| Additional component | Photopolymerization initiator | TPO | g | 30 | 30 | 30 | 30 | 30 | 30 |
| | Polymerization inhibitor | BHT | g | 5.0 | 5.0 | 5.0 | 5.0 | 5.0 | 5.0 |

| | | | | | | | | | |
|---|---|---|---|---|---|---|---|---|---|
| (* 1): (Meth)acrylic based polymerizable monomer component (A) (*2): Polyfunctional (meth)acrylic based polymerizable monomer (a-1) (*3): Monofunctional (meth)acrylic based polymerizable monomer (a-2) | | | | | | | | | |

### [Prosthetic Tooth containing Acrylic based Resin (II)]

"BV": resin tooth "Veracia (registered trademark)", available from SHOFU INC.
"BS": resin tooth "SURPASS (registered trademark)", available from GC Corporation

### [Adhesive for Denture Base]

"UNIFAST (registered trademark) III", available from GC Corporation, powder-liquid type adhesive of methyl methacrylate (MMA) and polymethyl methacrylate (PMMA)

### [Examples 1 to 9 and Comparative Examples 1 to 6]

As described below, the stereolithographic articles of the curable compositions (I) obtained in Reference Examples each were adhered to the prosthetic tooth containing the acrylic based resin (II) according to the combinations shown in Tables 2 and 3 below with the adhesives described below by the method shown later, so as to produce photocured test pieces, which were then evaluated for adhesiveness.

In Examples 1 to 6 and Comparative Examples 1 to 5, the compositions (III), which had the same composition as the curable composition (I) shown in Tables 2 and 3, respectively, each were used as the adhesive.

In Example 7, an adhesive composition obtained by diluting the composition (III), which had the same composition as the curable composition (I)-1 used, with ethyl acetate (available from FUJIFILM Wako Pure Chemical Corporation) to 30% by mass was used as the adhesive.

In Example 8, an adhesive composition obtained by diluting the composition (III), which had the same composition as the curable composition (I)-1 used, with ethyl acetate (available from FUJIFILM Wako Pure Chemical Corporation) to 15% by mass was used as the adhesive.

In Example 9, the stereolithographic article was obtained from a curable composition obtained by diluting the curable composition (I)-1 obtained in Reference Example 1 with PMMA (polymethyl methacrylate "Hi-pearl (registered trademark) D-250ML", available from Negami Chemical Industrial Co., Ltd.) to 40% by mass, and an adhesive composition obtained by diluting the composition (III), which had the same composition as the curable composition (I)-1 used, with ethyl acetate (available from FUJIFILM Wako Pure Chemical Corporation) to 15% by mass was used as the adhesive.

In Comparative Example 6, "UNIFAST (registered trademark) III" was used as the adhesive.

### <Adhesiveness of Denture Base and Prosthetic Tooth>

The curable compositions (I)-1, (I)-2, (I)-3, (I)-4, (I)-5, and (I)-6 each were subjected to stereolithography with a stereolithography machine (DigitalWax (registered trademark) 020D, available from DWS S.r.l.), so as to produce a test piece having a diameter of 5.0 mm and a height of 5.0 mm, which was flashed 2,000 times with a light irradiator (Otoflash (registered trademark) G171, available from Envision TEC, Inc.) to complete curing. Thereafter, one flat surface thereof was polished with silicon carbide paper #600 (available from Nihon Kenshi Co., Ltd.) under running water, and then water on the surface was blown off with a dental air syringe, so as to provide an adherend corresponding to a denture base.

Separately, the base surface of each of the resin teeth "BV" and "BS" was polished with silicon carbide paper #600 (available from Nihon Kenshi Co., Ltd.) to form a flat surface. According to the method B of JIS T6611:2009, the resin tooth was embedded in a stainless steel ring holder for mounting on the jig for shearing test by using a resin for dental impression tray (product name: "TRAY RESIN (registered trademark) II", available from SHOFU INC.) as the embedding material. At this time, the embedding material was charged after disposing the polished surface of the resin tooth on the lower bottom part of the stainless steel ring holder, and thereby the resin tooth was embedded in such a manner that the polished surface of the resin tooth was allowed to be in parallel to the contact surface of the stainless steel ring holder, and the resin tooth was fixed to expose from the embedding material. Thereafter, the resin tooth and the embedding material inside the stainless steel ring holder were polished with silicon carbide paper #600 (available from Nihon Kenshi Co., Ltd.) under running water. The polished surface of the resin tooth was polished to a height that was not higher than the contact surface of the stainless steel ring holder but was not higher by 1 mm or more than the surface of the embedding material.

After completing the polishing, water on the surface of the resin tooth was air blown off for drying, so as to provide an adherend corresponding to a prosthetic tooth.

According to the combination of each of the curable composition (I), the prosthetic teeth containing the acrylic based resin (II), and the adhesives of Examples and Comparative Examples, approximately 0.01 of the adhesive was coated on the polished surface of the resulting adherend corresponding to a denture base with a brush, on which the polished surface of the adherend corresponding to a prosthetic tooth under pressure, and after wiping off the excessive adhesive, the adhesive was flashed 1,000 times with a light irradiator ("Otoflash (registered trademark) G171", available from Envision TEC, Inc.). Five test pieces in total were produced, and each were stored in water at 37°C for one day, and then taken out from water. After wiping off water, by using a universal testing machine ("Autograph (registered trademark) AG-I 100kN", available from SHIMADZU CORPORATION), the stainless steel ring holder of the test piece was mounted on the jig for shearing test according to JIS T6611:2009, and a shearing force was applied in the direction in parallel to the adhesion surface between the prosthetic tooth and the test piece of the curable composition (I) having a diameter of 5.0 mm and a height of 5.0 mm, so as to measure the shearing adhesion strength. The shearing adhesion strength was measured at a crosshead speed of 1 mm/min. The average value of the measured values of the five test pieces was designated as the shearing adhesion strength.

In this test, an adhesion strength of 5 MPa or more is a clinically usable adhesion strength, that of 10 MPa or more is a good adhesion strength equivalent to the ordinary methods, and that of 15 MPa or more is a significantly excellent adhesion strength exceeding the ordinary methods. In the observation of the state of the interfaces of the test piece after the test, the case where any of the adherend corresponding to a denture base produced by stereolithography from the curable composition (I) and the adhesive was broken was evaluated as good adhesiveness "A", and the case where detachment occurred at any of the interfaces of the adherend corresponding to a denture base produced by stereolithography from the curable composition (I) and the prosthetic tooth containing the acrylic based resin (II) was evaluated as inferior adhesiveness "F".

The results are shown in Tables 2 and 3 below.

**Table 2**

| | | Unit | Example 1 | Example 2 | Example 3 | Example 4 | Example 5 | Example 6 | Example 7 | Example 8 | Example 9 |
|---|---|---|---|---|---|---|---|---|---|---|---|
| Curable composition (I) | (I)-1 | % by mass | 100 | - | - | - | - | - | 100 | 100 | 40 |
| | (I)-2 | % by mass | - | 100 | - | - | - | - | - | - | - |
| | (I)-3 | % by mass | - | - | 100 | - | - | - | - | - | - |
| | (I)-4 | % by mass | - | - | - | 100 | - | - | - | - | - |
| | (I)-5 | % by mass | - | - | - | - | 100 | - | - | - | - |
| | (I)-6 | % by mass | - | - | - | - | - | 100 | - | - | - |
| | PMMA (*5) | % by mass | - | - | - | - | - | - | - | - | 60 |
| Prosthetic tooth containing acrylic based resin (II) | | | BV | BS | BV | BV | BV | BV | BV | BV | BV |
| Adhesive composition | (I)-1 (*4) | % by mass | 100 | - | - | - | - | - | 30 | 15 | 30 |
| | (I)-2 (*4) | % by mass | - | 100 | - | - | - | - | - | - | - |
| | (I)-3 (*4) | % by mass | - | - | 100 | - | - | - | - | - | - |
| | (I)-4 (*4) | % by mass | - | - | - | 100 | - | - | - | - | - |
| | (I)-5 (*4) | % by mass | - | - | - | - | 100 | - | - | - | - |
| | (I)-6 (*4) | % by mass | - | - | - | - | - | 100 | - | - | - |
| | Ethyl acetate | % by mass | - | - | - | - | - | - | 70 | 85 | 70 |
| Evaluation | Adhesion strength | MPa | 16.7 | 16.1 | 12.5 | 11.4 | 9.1 | 12.6 | 12.8 | 12.0 | 7.8 |
| | State of interface | - | A | A | A | A | A | A | A | A | A |

| | | | | | | | | | | | |
|---|---|---|---|---|---|---|---|---|---|---|---|
| (*4): The number of the composition (I) having the same composition as the composition (III) contained in the adhesive composition is shown. (*5): PMMA (polymethyl methacrylate "Hi-pearl (registered trademark) D-250ML", available from Negami Chemical Industrial Co., Ltd.) | | | | | | | | | | | |

**Table 3**

| | | Unit | Comparative Example 1 | Comparative Example 2 | Comparative Example 3 | Comparative Example 4 | Comparative Example 5 | Comparative Example 6 |
|---|---|---|---|---|---|---|---|---|
| Curable composition (I) | (I)-1 | % by mass | 100 | - | - | - | - | 100 |
| | (I)-2 | % by mass | - | 100 | - | - | - | - |
| | (I)-3 | % by mass | - | - | 100 | - | - | - |
| | (I)-4 | % by mass | - | - | - | 100 | - | - |
| | (1)-5 | % by mass | - | - | - | - | 100 | - |
| Prosthetic tooth containing acrylic based resin (II) | | | BV | BV | BV | BV | BV | BV |
| Adhesive composition | (I)-1 (*4) | % by mass | - | 100 | - | - | - | - |
| | (I)-2 (*4) | % by mass | - | - | 100 | - | - | - |
| | (I)-3 (*4) | % by mass | - | - | - | 100 | - | - |
| | (I)-4 (*4) | % by mass | - | - | - | - | 100 | - |
| | (I)-5 (*4) | % by mass | 100 | - | - | - | - | - |
| | Unifast III | % by mass | - | - | - | - | - | 100 |
| Evaluation | Adhesion strength | MPa | 2.3 | 3.6 | 1.7 | 1.8 | 2.3 | 4.6 |
| | State of interface | - | F | F | F | F | F | F |

| | | | | | | | | |
|---|---|---|---|---|---|---|---|---|
| (*4): The number of the composition (I) having the same composition as the composition (III) contained in the adhesive composition is shown. | | | | | | | | |

As shown in Tables 2 and 3, it is confirmed that in the case where the adhesive compositions in Examples 1 to 9 are used, the adhesion strength between the adherend corresponding to a denture base produced from the curable composition (I) and the prosthetic tooth containing the acrylic based resin (II) is excellent as compared to the case where the adhesive compositions in Comparative Examples 1 to 6 are used.

## Claims

1. A method of producing a denture, comprising adhering
a denture base that is a stereolithographic article of a curable composition (I) containing a (meth)acrylic based polymerizable monomer component (A) containing a (meth)acrylic based polymerizable monomer (m) as a major monomer, and a photopolymerization initiator, and a prosthetic tooth containing an acrylic based resin (II),
with an adhesive composition containing a composition (III) containing a (meth)acrylic based polymerizable monomer component (C) containing the same (meth)acrylic based polymerizable monomer (m) as contained in the curable composition (I).

2. The method of producing a denture according to claim 1, wherein the (meth)acrylic based polymerizable monomer (m) is a polyfunctional (meth)acrylic based polymerizable monomer (a-1).

3. The method of producing a denture according to claim 2, wherein the polyfunctional (meth)acrylic based polymerizable monomer (a-1) is at least one kind selected from a urethanated (meth)acrylate ester compound and an aromatic compound based (meth)acrylate ester compound having no urethane bond.

4. The method of producing a denture according to any one of claims 1 to 3, wherein the (meth)acrylic based polymerizable monomer component (A) contains a monofunctional (meth)acrylic based polymerizable monomer (a-2).

5. The method of producing a denture according to any one of claims 1 to 4, wherein the (meth)acrylic based polymerizable monomer component (C) contains a monofunctional (meth)acrylic based polymerizable monomer (a-2).

6. The method of producing a denture according to claim 4 or 5, wherein the monofunctional (meth)acrylic based polymerizable monomer (a-2) is a (meth)acrylate ester based polymerizable monomer having an aromatic ring.

7. The method of producing a denture according to any one of claims 1 to 6, wherein the curable composition (I) has a content of the (meth)acrylic based polymerizable monomer (m) of 50% by mass or more based on 100% by mass of the (meth)acrylic based polymerizable monomer component (A).

8. The method of producing a denture according to any one of claims 1 to 7, wherein the composition (III) has a content of the (meth)acrylic based polymerizable monomer (m) of 50% by mass or more based on 100% by mass of the (meth)acrylic based polymerizable monomer component (C).

9. The method of producing a denture according to any one of claims 1 to 8, wherein the curable composition (I) contains the (meth)acrylic based polymerizable monomer component (A) in an amount of 40% by mass or more based on 100% by mass of the curable composition (I).

10. The method of producing a denture according to any one of claims 1 to 9, wherein the composition (III) contains the (meth)acrylic based polymerizable monomer (C) in an amount of 40% by mass or more based on 100% by mass of the composition (III).

11. The method of producing a denture according to any one of claims 1 to 10, wherein the method comprises the following steps (1) and (2) in this order:
step (1): a step of coating the adhesive composition on at least one selected from the denture base and the prosthetic tooth, and
step (2): a step of adhering the denture base and the prosthetic tooth via the adhesive composition under pressure.

12. The method of producing a denture according to any one of claims 1 to 11, wherein the method comprises irradiating the adhesive composition with an active energy light ray, so that the adhesive composition is cured to adhere the denture base and the prosthetic tooth.
